# EUROPÄISCHE PATENTSCHRIFT

(11) **EP 3 713 624 B1**
(45) Veröffentlichungstag und Bekanntmachung des Hinweises auf die Patenterteilung: **04.08.2021**
(21) Anmeldenummer: 18811359.1
(22) Anmeldetag: 13.11.2018
(51) Int. Cl.: A61M 5/315, A61M 5/20, A61M 5/32

(54) **VERZÖGERUNGSEINRICHTUNG FÜR EIN MEDIZINISCHES VERABREICHUNGSGERÄT**
DELAY MECHANISM FOR A MEDICAL DISPENSING DEVICE
MECANISME DE RETARDEMENT POUR UN DISPOSITIF DE DISTRIBUTION MEDICALE

(30) Priorität: 21.11.2017 CH 14112017
(43) Veröffentlichungstag der Anmeldung: 30.09.2020
(73) Patentinhaber: Ypsomed AG, 3401 Burgdorf (CH)
(72) Erfinder: FIECHTER, Marc, 3510 Konolfingen (CH); BURREN, Stefan, 3150 Schwarzenburg (CH); TSCHIRREN, Markus, 3400 Burgdorf (CH); HUNZIKER, Markus, 4460 Gelterkinden (CH)
(74) Vertreter: Kleiner, Stefan
(86) Internationale Anmeldenummer: PCT/IB2018/058902
(87) Internationale Veröffentlichungsnummer: WO 2019/102300

(56) Entgegenhaltungen:
- WO-A1-2016/205962
- WO-A2-2012/032411
- WO-A2-2016/034407
- CH-A2- 712 459
- US-A1- 2017 087 304

## Beschreibung

### TECHNISCHES GEBIET

Die vorliegende Erfindung betrifft das Gebiet der medizinischen Verabreichungsvorrichtungen zur Verabreichung von flüssigen Substanzen, insbesondere Insulin- und Hormonpräparationen. Die Erfindung bezieht sich auf eine Kolbenstangeneinheit für eine medizinische Verabreichungsvorrichtung. Die Kolbenstangeneinheit umfasst eine äussere und eine innere Kolbenstange. Die innere Kolbenstange wirkt mit einem Fluidverdrängungselement zusammen, welches sich in einer Kavität mit einem dickflüssigen Fluid befindet, so dass bei einer relativen Bewegung zwischen dem Fluidverdrängungselement und der äusseren Kolbenstange durch Verdrängen des Fluids ein Bewegungswiderstand entsteht, wodurch ein Auslösen eines Mechanismus der Verabreichungsvorrichtung verzögerbar ist.

### HINTERGRUND

Bei medizinischen Verabreichungsvorrichtungen kann es wünschenswert sein, dass bestimmte Funktionen verzögerbar sind. So kann es beispielsweise bei einem Injektionsvorgang notwendig sein, dass nach der Injektion der medizinischen Substanz wie beispielsweise Insulin die Injektionsnadel nicht sofort herausgezogen wird, da sonst die injizierte Flüssigkeit aus der Injektionsstelle austritt. In Folge kommt es zu einer Unterdosierung. Zudem brauchen bestimmte medizinische Substanzen, insbesondere zähflüssige Medikamente eine bestimmte Zeitdauer bis sie im Körper des Patienten angekommen sind. Aus diesen Gründen wird ein abschliessender Vorgang wie beispielsweise ein Nadelrückzugmechanismus oder ein Endsignal wie zum Beispiel ein Signalton verzögert, so dass der Anwender den Verabreichungsvorgang nicht zu früh beendet und die Nadel nicht zu früh herauszieht.

Aus der Patentschrift EP 2542280 B1 ist ein Autoinjektor bekannt, bei welchem die Spritze nach der Injektion zurück in den Autoinjektor gezogen wird. Der Rückzugsmechanismus wird ausgelöst, nachdem eine Kolbenstange den Stopfen der vorbefüllten Spritze um eine gewisse Distanz verschoben hat. Der Spritzenrückzug wird dabei nicht unmittelbar nach dem Erreichen der gewissen Distanz vollführt, sondern erst nach einer bestimmten Verzögerung. Unmittelbar nach dem Erreichen der gewissen Distanz wird durch die Bewegung der Kolbenstange ein Freigabeteil freigegeben, welches sich durch die Wirkung einer vorgespannten Torsionsfeder zu drehen beginnt. Erst wenn sich das Freigabeteil um einen bestimmten Winkel gedreht hat, wird der Spritzenrückzug freigegeben. Die Drehung des Freigabeteils erzeugt also eine Verzögerung beim Spritzenrückzug. In der Schrift wird das Auslösen des Spritzenrückzugs weiter verzögert, indem die Rotation des Freigabeteils durch eine viskose Substanz gebremst wird.

Die Patentanmeldung US 2017/0087304 A1 beschreibt eine Verzögerungseinrichtung eines Injektors, um ein akustisches oder taktiles Endsignal zu verzögern, wenn eine medizinische Substanz vollständig injiziert wurde. Die Verzögerungseinrichtung umfasst einen Kolbenstangenantrieb, welcher ein rohrförmiges Gehäuse aufweist, ein in diesem Gehäuse drehbar gelagertes hülsenförmiges Verzögerungselement sowie ein koaxial im Verzögerungselement gelagertes zylinderförmiges Signalerzeugungselement. Das Signalerzeugungselement wirkt dabei mit einer Kolbenstange zusammen, welche auf einen Behälter mit dem Medikament wirkt. Am Ende einer Injektion wird das Verzögerungselement durch eine Kraft vom Kolbenstangenantrieb relativ zu diesem und relativ zum Signalerzeugungselement in eine Rotation versetzt. Zwischen der Innenfläche des Gehäuses des Kolbenstangenantriebs und der Hülsenaussenfläche des Verzögerungselements und zwischen der Hülseninnenfläche und der Aussenfläche des Signalerzeugungselements befindet sich Fett, welches die Drehbewegung des Verzögerungselements dämpft und dadurch das Endsignal vom Signalerzeugungselement verzögert.

Einen weiteren Verzögerungsmechanismus offenbart die WO 2016/205962 A1, bei der am Ende einer Injektion ein Freigabemechanismus, welcher das Ende der Injektion anzeigt, verzögert wird. Hierzu umfasst der Injektor eine äussere Kolbenstange mit einer Kavität und einer Bohrung und eine in der Bohrung gelagerte innere Kolbenstange. Die innere Kolbenstange ist mit einem ruderartigen Element verbunden, welches in die Kavität ragt. Diese ist mit einem viskosen Fluid gefüllt. Während der Injektion werden über einen Antrieb die äussere und die innere Kolbenstange zusammen in axialer Richtung bewegt und drücken auf einen Stopfen eines mit einer medizinischen Substanz gefüllten Behälters damit die medizinische Substanz ausgeschüttet wird. Am Ende der Injektion löst sich die innere Kolbenstange aus einer Führung, so dass sie sich relativ zur äusseren Kolbenstange drehen kann. Die Drehbewegung der inneren Kolbenstange wird dabei durch das ruderartige Element gedämpft, da dieses aufgrund des Fluids in der Kavität einen Widerstand erfährt. Dadurch wird der Freigabemechanismus verzögert.

CH 712 459 A2 offenbart eine innere sowie eine äußere Kolbenstange und ein Fluidverdrängungselement.

Solche bekannten Verzögerungseinrichtungen haben den Nachteil, dass sie aufwändig herzustellen sind. Das dickflüssige Fluid muss während der Montage auf der Montageeinrichtung für die medizinische Verabreichungsvorrichtung eingefüllt werden. Da der Herstellungsprozess für die Verabreichungsvorrichtung hohen Anforderungen bezüglich partikulärer und mikrobiologischer Sauberkeit genügen muss, ist das Abfüllen des dickflüssigen Fluids äusserst heikel und aufwändig zu realisieren.

Es ist Aufgabe der Erfindung eine Verabreichungsvorrichtung mit einer Verzögerungseinrichtung zu schaffen, die einfach konstruierbar und herstellbar ist.

### DARSTELLUNG DER ERFINDUNG

Die Erfindung ist bestimmt durch den Gegenstand von Anspruch 1 sowie von Anspruch 14, wobei weitere Ausführungsformen in den abhängigen Ansprüchen bestimmt sind. Die Aufgabe wird durch die Merkmale des Anspruchs 1 gelöst. Gemäss Anspruch 1 ist eine äussere Kolbenstange zweistückig ausgebildet mit einem distalen, ersten Kolbenstangenteil, welcher eine Kontaktfläche zum Zusammenwirken mit einem Stopfen zum Ausschütten der Substanz aus dem Produktbehälter umfasst und wobei der erste Kolbenstangenteil eine mit einem dickflüssigen Fluid befüllbare Kavität bildet, und einem an den ersten Kolbenstangenteil anschliessenden zweiten Kolbenstangenteil. Bevorzugte Ausführungsformen sind Gegenstand der abhängigen Ansprüche.

Indem die äussere Kolbenstange zweistückig ausgeführt ist, kann bei der Montage der distale, erste Kolbenstangenteil mit dem dickflüssigen Fluid und dem Fluidverdrängungselement räumlich getrennt vom zweiten Kolbenstangenteil vormontiert werden. Der erste Kolbenstangenteil kann in seiner Grösse auf das Fluidverdrängungselement angepasst werden. Dadurch kann der erste Kolbenstangenteil mit dem dickflüssigen Fluid zu einer kompakten Einheit vormontiert werden, welche gut handhabbar ist. Durch diese Vormontage kann die Gefahr einer Kontamination der übrigen Bestandteile der Verabreichungsvorrichtung mit dem Fluid reduziert werden. Vorzugsweise wird der erste Kolbenstangenteil mit dem Fluidverdrängungselement und einem allenfalls vorhandenen Dichtelement zu einer gegenüber dem abgefüllten Fluid dichten Einheit vormontiert, so dass das Fluid nicht aus der Kavität auslaufen kann. Diese kompakte, abgedichtete Einheit kann anschliessend in die Montageumgebung des zweiten Kolbenstangenteils und den übrigen Bestandteilen der Verabreichungsvorrichtung gebracht werden. Das vereinfacht die Montage der Kolbenstangeneinheit. Eine Befüllung der äusseren Kolbenstange mit einem hochviskosen, dickflüssigen Fluid erfolgt zur Vermeidung eines Einschlusses von Luftbläschen bevorzugt unter Vakuum. Ebenso resultiert ein dichter Abschluss der Kavität unter Vakuum in einem Unterdruck, welcher zusätzlich zur Dichtung beiträgt. Dieser Abfüllprozess ist bedeutend platzsparender und einfacher zu bewerkstelligen mit einem ersten Kolbenstangenteil, welcher in seiner Grösse auf das Volumen des Fluids angepasst ist, als mit einer einstückigen äusseren Kolbenstange, bei welcher nur ein vergleichsweise kleines Volumen eingebracht wird. Durch den zusätzlichen Prozessschritt des Abfüllen und Abdichten unter Vakuum werden erfindungsgemäss kleinstmögliche Einheiten hergestellt, welche vor der weiteren Montage separat gereinigt und geprüft werden können.

Zudem ermöglicht die erfindungsgemässe zweistückige äussere Kolbenstange, dass die beiden Kolbenstangenteile je aus einem unterschiedlichen Material hergestellt sein können. Dadurch kann die Kolbenstangeneinheit im Bereich des ersten Kolbenstangenteils und im Bereich des zweiten Kolbenstangenteils unterschiedliche physikalische Eigenschaften aufweisen, welche für den jeweiligen Bereich besonders vorteilhaft sind. So kann beispielsweise der erste Kolbenstangenteil aus einem Kunststoff gefertigt werden, welcher eine besondere chemische Beständigkeit aufweist und sich somit besonders gut für das Zusammenwirken mit einem dickflüssigen Fluid eignet, während der zweite Kolbenstangenteil beispielsweise aus einem Kunststoffteil gefertigt werden kann, welcher in Bezug auf die Herstellung und Montage vorteilhaft ist oder spezielle Gleiteigenschaften aufweist, damit die innere Kolbenstange besonders gut bewegbar gelagert werden kann. Das bedeutet, die Kolbenstangeneinheit kann mit der zweistückigen äusseren Kolbenstange so konstruiert werden, dass sie die jeweiligen örtlichen Anforderungen besonders gut erfüllt.

Der Schutz umfasst zudem auch die Einzelteile der Kolbenstangeneinheit, welche zur Kolbenstangeneinheit zusammengebaut werden können. Die Kolbenstangeneinheit umfasst eine äussere Kolbenstange mit einer Kontaktfläche an einem distalen Ende, wobei die äussere Kolbenstange zweistückig ausgebildet ist mit einem distalen, ersten Kolbenstangenteil, welcher die Kontaktfläche umfasst, eine Kavität bildet, und mittels einer Dichtung und einem Fluidverdrängungselement fluiddicht abschliessbar ist, und einem an den ersten Kolbenstangenteil anschliessbaren zweiten Kolbenstangenteil. Die Kolbenstangeneinheit umfasst weiter eine innere Kolbenstange, die derart ausgebildet ist, dass sie in der äusseren Kolbenstange gelagert werden kann, ein Fluidverdrängungselement zum Verdrängen eines Fluids in der Kavität und eine Dichtung zum Abdichten zwischen dem Fluidverdrängungselement und dem ersten Kolbenstangenteil.

Die Angabe "distal" bezieht sich in der vorliegenden Beschreibung auf eine Richtung zum einstechseitigen Ende der Verabreichungsvorrichtung hin. Demgegenüber bezieht sich die Angabe "proximal" auf eine Richtung zum hinteren, dem einstechseitigen Ende gegenüberliegenden Ende der Verabreichungsvorrichtung hin.Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

In der vorliegenden Beschreibung wird unter dem Begriff "Verabreichungsvorrichtung" eine Vorrichtung verstanden, mit der eine medizinische Substanz aus einem Produktbehälter injiziert werden kann. Die Injektion kann beispielsweise intrakutan, subkutan oder intramuskulär erfolgen. Die Verabreichungsvorrichtung kann demnach beispielsweise als Einweg-Injektionssystem zur einmaligen, manuellen oder bevorzugt automatischen Abgabe einer voreingestellten, bevorzugt maximalen Dosis aus dem Produktbehälter ausgebildet sein. Ein Einweg-Injektionssystem ist ein System, welches dazu verwendet wird, die in einer nicht nachfüllbaren und nicht austauschbaren Karpule befindliche medizinische Substanz zu injizieren. Ist die zur Injektion vorgesehene Menge der medizinischen Substanz in einem oder in mehreren Injektionsvorgängen injiziert worden, wird das Einweg-Injektionssystem ausgewechselt. Die Karpule im Einweg-Injektionssystem kann nicht ausgetauscht werden. Demgegenüber ist bei einem Mehrweg-Injektionssystem die Karpule mit der medizinischen Substanz austauschbar.

Gemäss der Erfindung ist eine den Mechanismus auslösende Auslöseposition der Verabreichungsvorrichtung verzögert erreichbar. Ein solcher Mechanismus ermöglicht vorzugsweise eine Funktion der Verabreichungsvorrichtung, welche am Ende eines Verabreichungsvorgangs ausgeführt wird. So kann der Mechanismus zum Beispiel ein akustisches, optisches oder taktiles Endsignal ausgeben, um dem Anwender das Ende des Verabreichungsvorgangs anzuzeigen. Ein akustisches Endsignal kann beispielsweise ein Piepston oder ein mechanisches Klickgeräusch sein. Ein optisches Endsignal kann beispielsweise durch ein Erscheinen eines Anzeigeelements in einem Fenster in der Verabreichungsvorrichtung, durch einen Farbwechsel eines Elements oder durch eine Formänderung eines Elements gebildet werden. Ein taktiles Endsignal kann in Form einer durch den Anwender spürbaren Vibration, eines Schlags oder einer Formänderung im Griffbereich der Verabreichungsvorrichtung realisiert sein. Jedoch kann der Mechanismus zum Beispiel auch eine aus dem Stand der Technik bekannte Nadelrückziehvorrichtung umfassen, welche eine Nadel nach einem Verabreichungsvorgang aus der Haut herauszieht und in die Verabreichungsvorrichtung einfährt.

Unter dem Begriff "Kolbenstange" wird eine Vorrichtung verstanden, welche eine physische Verbindung zwischen einer Antriebseinheit und einem Produktbehälter herstellt und mit der eine Kraft von der Antriebseinheit auf einen Stopfen im Produktbehälter übertragen werden kann.

In einer bevorzugten Ausführung hat die Kolbenstangen eine längliche Gestalt. Vorzugsweise ist sie im Wesentlichen zylinderförmig ausgebildet. Jedoch muss die Kolbenstange nicht zwingend einen kreisrunden Querschnitt aufweisen. Der Querschnitt kann zum Beispiel auch rechteckförmig, polygonförmig oder eine elliptische Form aufweisen. Vorzugsweise ist die gesamte Kolbenstangeneinheit innerhalb eines Gehäuses der Verabreichungsvorrichtung relativ zum Gehäuse und in Längsrichtung des Gehäuses verschiebbar. Dadurch kann der Stopfen des Produktbehälters zum Ausschütten des Produkts aus dem Produktbehälter in Längsrichtung verschoben werden.

Die äussere und die innere Kolbenstange sind zwei einzelne Teile, welche getrennt und unabhängig voneinander herstellbar sind. Im montierten Zustand können die innere und die äussere Kolbenstange lösbar oder unlösbar miteinander verbunden sein. Ebenso sind der erste und der zweite Kolbenstangenteil einzelne Teile, welche einzeln und getrennt voneinander herstellbar sind. Im montierten Zustand der Kolbenstangeneinheit sind der erste und der zweite Kolbenstangenteil lösbar oder unlösbar miteinander verbunden.

Die äussere Kolbenstange weist einen Hohlraum auf, welcher durch die miteinander verbundenen äusseren Kolbenstangenteile gebildet wird. Der Hohlraum ist mindestens abschnittsweise im Wesentlichen zylinderförmig ausgebildet und weist eine Öffnung an einem proximalen Ende des zweiten Kolbenstangenteils auf. Ein solcher Hohlraum kann auch als Sacklochbohrung bezeichnet werden, wobei die Herstellung keineswegs auf ein Bohren beschränkt ist. Bevorzugt bildet der erste Kolbenstangenteil den Abschluss des Hohlraums. Hierzu weist der erste Kolbenstangenteil eine Kavität auf. Diese ist vorzugsweise in mehrere Kammern unterteilt. In alternativen Ausführungen kann die Kavität jedoch auch zylinderförmig, rechteckförmig, halbkugelförmig oder schalenförmig im ersten Kolbenstangenteil ausgebildet sein. Der zweite Kolbenstangenteil weist vorzugsweise eine hülsenförmige Gestalt auf, wodurch der zweite Kolbenstangenteil den zylinderförmigen Abschnitt des Hohlraums der äusseren Kolbenstange bildet.

In einer bevorzugten Ausführung ist das Fluidverdrängungselement als einzelnes Teil ausgebildet, welches mit der inneren Kolbenstange lösbar, beispielsweise mittels eines lösbaren Schnappverschlusses oder Schraubverbindung, oder unlösbar, zum Beispiel mittels Verkleben oder Verschweissen verbunden werden kann. Das bedeutet, dass das Fluidverdrängungselement nicht einstückig an der inneren Kolbenstange ausgebildet ist. In einer alternativen Ausführung kann das Fluidverdrängungselement nicht als einzelnes Teil, sondern als Auskragung, Flügel oder Vorstehung auf der inneren Kolbenstange ausgebildet sein. In diesem Fall ist das Fluidverdrängungselement einstückig in die innere Kolbenstange integriert und damit kein einzelnes von der inneren Kolbenstange getrennt herstellbares Teil.

Das Fluid ist vorzugsweise eine nicht-flüchtige Flüssigkeit und kann zum Beispiel eine einfache Newton'sche Flüssigkeit sein. Die Verzögerung kann über die Viskosität der Flüssigkeit eingestellt werden.

Die Kolbenstangeneinheit umfasst weiter eine Dichtung zum fluidischen Abdichten der Kavität. Mit der Dichtung kann die Gefahr eines Auslaufens des Fluids aus der Kavität reduziert werden. Die Dichtung kann zum Beispiel in Form eines Dichtelements wie beispielsweise einem Dichtring oder aber durch eine präzise Passung eines Aussenmasses des Fluidverdrängungselements zu einem Innenmass des ersten Kolbenstangenteils gebildet werden. Die Dichtung muss also nicht zwingend als eigenständiges Bauteil ausgeführt sein. Vorzugsweise ist die Dichtung ein Dichtelement in der Form eines O-Rings, der in einem rotationssymmetrischen Bereich des Fluidverdrängungselements angeordnet ist.

In einer bevorzugten Ausführung ist die innere Kolbenstange in Längsrichtung der Kolbenstangeneinheit relativ zur äusseren Kolbenstange axialfest. Das bedeutet, wenn die innere Kolbenstange durch eine Antriebseinheit in Längsrichtung relativ zu einem Gehäuse der Verabreichungsvorrichtung bewegt wird, um den Stopfen des Produktbehälters zu verschieben, um das Produkt auszuschütten, wird auch die äussere Kolbenstange, also der erste und der zweite Kolbenstangenteil, mitbewegt.

Vorzugsweise ist die äussere Kolbenstange durch eine formschlüssige Verbindung an der inneren Kolbenstange gehalten, so dass die äussere Kolbenstange in Längsrichtung nicht relativ zur inneren Kolbenstange verschiebbar ist. Hierzu weist die innere Kolbenstange bevorzugt in radialer Richtung eine Auskragung, beispielsweise in der Form eines Nockens, einer Nase oder eines umlaufenden Kragens, auf. Diese Auskragung kann dann mit einer Abschlussfläche, einem Absatz oder einer Ausnehmung in der äusseren Kolbenstange zusammenwirken, so dass die innere Kolbenstange in Längsrichtung zur äusseren Kolbenstange unbewegbar gelagert ist. Alternativ dazu kann aber auch die äussere Kolbenstange eine Auskragung und die innere Kolbenstange ein komplementäres Element aufweisen.

Vorzugsweise ist die äussere Kolbenstange derart ausgestaltet, dass mindestens die Hälfte der Länge der inneren Kolbenstangen in der äusseren Kolbenstange aufgenommen ist. Dadurch kann die Kolbenstangeneinheit kompakt konstruiert werden. In einer bevorzugten Ausführung entspricht die Länge des ersten Kolbenstangenteils im Wesentlichen der Länge des Fluidverdrängungselements, so dass das Fluidverdrängungselement grösstenteils im ersten Kolbenstangenteil aufgenommen werden kann. Weiter entspricht vorzugsweise die Länge des zweiten Kolbenstangenteils im Wesentlichen der Länge der inneren Kolbenstange ohne Fluidverdrängungselement, so dass die innere Kolbenstange ohne Fluidverdrängungselement grösstenteils vom zweiten Kolbenstangenteil aufgenommen werden kann.

Vorzugsweise ist das Fluidverdrängungselement bei der Verzögerungsbewegung um eine Längsachse der äusseren Kolbenstange relativ zur äusseren Kolbenstange drehbar. Durch eine Drehung des Fluidverdrängungselement relativ zur äusseren Kolbenstange wird das in der Kavität befindliche Fluid verdrängt, wodurch der Bewegungswiderstand und somit die gewünschte Verzögerung entsteht. Im Unterschied zu einem in Längsrichtung der Kolbenstangeneinheit verschiebbaren Fluidverdrängungselement, benötigt eine Konstruktion mit einem drehbaren Fluidverdrängungselement weniger Platz und die Kolbenstangeneinheit kann kompakt konstruiert werden.

In einer bevorzugten Ausführung wird beim Drehen des Fluidverdrängungselements das Fluid in Umfangrichtung und in radialer Richtung verdrängt. Alternativ kann, bei einer entsprechenden Ausbildung des Fluidverdrängungselements, zum Beispiel mit einer angewinkelten Fläche, das Fluid mehrheitlich in Längsrichtung verdrängt werden.

In einer vorteilhaften Ausführung ist das Fluidverdrängungselement um einen Winkel von mindestens 60°, vorzugsweise mindestens 90° und besonders bevorzugt zwischen 100° und 120° relativ zur äusseren Kolbenstange drehbar. Der Drehwinkel ist abhängig von der Form des Fluidverdrängungselements und der Ausbildung der Kavität. Je grösser der Drehwinkel ist, desto länger kann das Auslösen des Mechanismus verzögert werden. Somit kann durch die Wahl der Form des Fluidverdrängungselement und der Kavität im ersten Kolbenstangenteil die Verzögerungszeit eingestellt werden. Wie oben erwähnt, ist ausserdem die Verzögerungszeit durch die Viskosität des Fluids einstellbar.

In einer alternativen Ausführung besteht die Möglichkeit, dass das Fluidverdrängungselement weniger als 60° drehbar ist. Die Verzögerung der Auslösung des Mechanismus der Verabreichungsvorrichtung ist in diesem Fall entsprechend kürzer.

Bevorzugt weist das Fluidverdrängungselement einen Zapfen auf zum Übertragen von axialen Kräften und zum Zentrieren des Fluidverdrängungselements in der Kavität. Vorzugsweise dient der Zapfen zur Übertragung von axialen Kräften für das Ausschütten der medizinischen Substanz. Dabei werden die axialen Kräfte von der inneren Kolbenstange auf das Fluidverdrängungselement übertragen. Dieses überträgt die axialen Kräfte mittels des Zapfens weiter auf den ersten äusseren Kolbenstangenteil, von dem sie auf den Stopfen der Karpule übertagen werden. Es hat sich gezeigt, dass mit dieser Kraftübertragung über den Zapfen unerwünschte Reibungskräfte bei der Bewegung des Fluidverdrängungselements möglichst gering gehalten werden können.

Mittels des Zapfens kann ein Verbiegen oder Verschieben des Fluidverdrängungselements aufgrund von Kräften, die beim Verdrängen des Fluids entstehen, vermieden werden. Mit Vorteil ist der Zapfen als zylindrischer Stift am distalen Ende des Fluidverbindungselements ausgebildet und ist in einer Aufnahme in der Kavität im ersten Kolbenstangenteil aufgenommen. Bei einer Drehung des Fluidverdrängungselements ist dieses durch den Zapfen in der Kavität zentriert und das Fluidverdrängungselement wird durch den Zapfen geführt. Damit kann dieses durch die beim Verdrängen des Fluids entstehenden radialen Kräften nicht verbogen werden und nicht verkanten.

Vorzugsweise weist das Fluidverdrängungselement mindestens zwei Flügel auf zum Verdrängen des Fluids in der Kavität. Mit den mindestens zwei Flügeln kann das dickflüssige Fluid besonders gut verdrängt werden, so dass der gewünschte Bewegungswiderstand und damit die Verzögerung effektiv erzeugt werden kann. Mit Vorteil stehen die Flügel in radialer Richtung von einem Mittelteil des Fluidverdrängungselements ab. In einer bevorzugten Ausführung sind die mindestens zwei Flügel in Drehrichtung um 180° versetzt zueinander angeordnet.

Alternativ besteht die Möglichkeit, dass das Fluidverdrängungselement keine Flügel umfasst, sondern im Querschnitt zum Beispiel eine elliptische Form aufweist und das Fluid beim Drehen des Fluidverdrängungselements durch diese Form verdrängt wird. Zudem besteht auch die Möglichkeit, dass das Fluidverdrängungselement einen kreisrunden Querschnitt hat und das freie Volumen der Kavität nur als dünner Spalt insbesondere als Ringspalt zwischen einer Innenfläche des ersten Kolbenstangenteils und dem Fluidverdrängungselement vorliegt. Der Bewegungswiderstand wird in diesem Fall nur durch den Scherwiderstand innerhalb des Fluids erzeugt.

Mit Vorteil weist die Kavität eine erste Kammer zum Aufnehmen eines ersten der mindestens zwei Flügel und eine zweite Kammer zum Aufnehmen des zweiten der mindestens zwei Flügel auf. Durch die Unterteilung der Kavität in zwei Kammern ist das Volumen, in dem sich das Fluid bewegen kann, eingeschränkt, so dass ein effektiver Bewegungswiderstand entsteht, wenn sich die Flügel des Fluidverdrängungselement je innerhalb einer Kammer bewegen.

Mit Vorteil weist das Fluid eine kinematische Viskosität zwischen 100'000 bis 500'000 Centistokes auf. Mit einer kinematischen Viskosität in diesem Bereich ist das Fluid genügend zähflüssig damit effektiv ein Bewegungswiderstand erzielt werden kann, um die Auslösung des Mechanismus entsprechend verzögern zu können. Über die Eigenschaften des Fluids lässt sich die Dauer der Verzögerung einstellen. Bevorzugt ist das Fluid eine Newton'sche Flüssigkeiten, insbesondere eine flüssige Silikonformulierung.

Das Fluid kann jedoch auch eine nicht Newton'sche Flüssigkeiten, insbesondere eine Flüssigkeit mit thixotropen Eigenschaften sein. Darunter ist eine Flüssigkeit zu verstehen, die unter Belastung zeitabhängig ihre Viskosität ändert. Das heisst, bei einer thixotropen Flüssigkeit reduziert sich die Viskosität zeitabhängig. Thixotrope Flüssigkeiten können im Ruhezustand sehr zäh sein und sich allenfalls ähnlich zu Feststoffen verhalten. Unter einer Scherlast, verringert sich die Viskosität unter Umständen zeitabhängig erheblich. Dem Fachmann sind eine Vielzahl thixotroper Flüssigkeiten zugänglich, insbesondere kieselgelhaltige oder hyaluronsäurehaltige Flüssigkeiten. Eine gute Alternative zu thixotropen Flüssigkeiten stellen strukturviskose Flüssigkeiten dar, welche wie thixotrope Flüssigkeiten im Ruhezustand eine erhöhte Viskosität aufweisen. Anders als bei thixotropen Flüssigkeiten reduziert sich bei strukturviskosen Flüssigkeiten die Viskosität mit steigender Schergeschwindigkeit und nicht abhängig von der Zeit. Beispielhaft seien hier Polymerlösungen oder lonomere genannt, welche die gewünschten Eigenschaften haben können. In einer weiteren Ausführung kann das Fluid jedoch auch eine anti-thixotrope (rheopexe) Flüssigkeit sein, bei der die Viskosität bei mechanischer Beanspruchung zeitabhängig zunimmt.

In einer bevorzugten Ausführung ist das Fluidverdrängungselement derart ausgebildet, dass zwischen dem Fluidverdrängungselement und einer Innenfläche des ersten Kolbenstangenteils ein exklusiver Spalt besteht, so dass bei einer relativen Bewegung zwischen Fluidverdrängungselement und äusserer Kolbenstange das Fluid durch den Spalt gezwungen wird. Der Spalt hat eine über seine Länge annähernd konstante lichte Weite von einigen Millimeter. In einer alternativen Ausführung kann der Spalt auch eine Weite von weniger als einem Millimeter aufweisen. Die Weite des Spalts kann je nach gewünschtem Bewegungswiderstand dimensioniert werden, um die Verzögerungszeit einzustellen. Zudem kann die Weite des Spalts an die Fliesseigenschaften des Fluids angepasst werden.

Falls das Fluidverdrängungselement relativ zur äusseren Kolbenstange drehbar gelagert ist, befindet sich der Spalt vorzugsweise in radialer Richtung zwischen einer Aussenfläche des Fluidverdrängungselements, insbesondere zwischen einer Aussenfläche eines Flügels und einer Innenwandung des ersten Kolbenstangenteils. Wenn das Fluidverdrängungselement relativ zur Innenwandung bewegt wird, wird das Fluid in der Kavität verdrängt und kann im Spalt von einer Seite des Fluidverdrängungselements zur anderen Seite fliessen. Somit kann dank des Spalts das Fluidverdrängungselement nicht blockieren, wenn es innerhalb der Kavität bewegt wird.

Alternativ dazu besteht auch die Möglichkeit, dass kein Spalt vorhanden ist. Das Fluid wird in diesem Fall unter Druck gesetzt oder es kann zum Beispiel durch eine Öffnung im Fluidverdrängungselement von einer Seite zur anderen fliessen.

Vorzugsweise ist die Kavität fluiddicht ausgebildet, so dass das Fluid während der relativen Bewegung zwischen Fluidverdrängungselement und äusserer Kolbenstange in der Kavität verbleibt. Gemäss der Erfindung wird die Verzögerung durch das Verdrängen des dickflüssigen Fluids in der Kavität des ersten Kolbenstangenteils erreicht. Dabei verbleibt das Fluid vorzugsweise innerhalb der Kavität, so dass durch die Bewegung des Fluidverdrängungselements das Fluid beliebig oft in der Kavität verdrängt werden kann und dadurch jedes Mal ein Bewegungswiderstand erzeugbar ist. Falls die Kavität in mehrere Kammern unterteilt ist, verbleibt das Fluid bei der Bewegung des Fluidverdrängungselements vorzugsweise in diesen Kammern. Dabei kann das Fluid bei der Bewegung beispielsweise von einer ersten Kammer in eine zweite Kammer fliessen ohne jedoch die Kammern zu verlassen.

In einer alternativen Ausführung kann das Fluid durch die Bewegung des Fluidverdrängungselements aus der Kavität der äusseren Kolbenstange gedrängt werden. In diesem Fall kann die Auslöseposition nur einmal verzögert erreicht werden.

Die Erfindung betrifft weiter eine Verzögerungseinheit für die medizinische Verabreichungsvorrichtung. Die Verabreichungsvorrichtung weist ein zum Ausschütten der medizinischen Substanz durch einen Antrieb bewegbares Ausschüttelement auf. Die Verzögerungseinheit umfasst ein in die Verabreichungsvorrichtung einführbaren, mit einem dickflüssigen Fluid zumindest näherungsweise vollständig und beispielsweise unter Vakuum befüllten Behälter, ein gegenüber dem Behälter in einer Verzögerungsrichtung bewegbares und an das Ausschüttelement ankoppelbares Fluidverdrängungselement, und eine Dichtung zwischen Behälter und Fluidverdrängungselement zum dichten Abschluss des dickflüssigen Fluids. Dabei ist die Verzögerungseinheit derart in der medizinischen Verabreichungsvorrichtung montierbar, dass gegen das Ende einer Ausschüttbewegung des Ausschüttelements das Fluidverdrängungselement und das angekoppelte Ausschüttelement sich in Verzögerungsrichtung in eine Auslöseposition bewegen.

In einer bevorzugten Ausführung ist das bewegbare Ausschüttelement als eine innere Kolbenstange ausgeführt, welche sich in einer äusseren Kolbenstange befindet. Die äussere Kolbenstange ist vorzugsweise zweistückig ausgebildet mit einem distalen, ersten Kolbenstangenteil, welcher als Behälter ausgebildet ist und einem an den ersten Kolbenstangenteil anschliessenden zweiten Kolbenstangenteil. In der Auslöseposition des Fluidverdrängungselement und des Ausschüttelements wird, wie weiter oben erwähnt, ein Mechanismus zum Erzeugen eines Endsignals oder ein Mechanismus zum Zurückziehen einer Nadel ausgelöst.

Ferner betrifft die Erfindung eine Verabreichungsvorrichtung zur Verabreichung einer medizinischen Substanz umfassend einen Signalerzeugungsmechanismus zum Erzeugen eines Signals und eine Kolbenstangeneinheit nach einem der Ansprüche 1 bis 11, wobei mit der Kolbenstangeneinheit am Ende eines Verabreichungsvorgangs das Erzeugen eines Signals mit dem Signalerzeugungsmechanismus verzögerbar ist.

Der Signalerzeugungsmechanismus kann ein Signal generieren, welches dem Benutzer anzeigt, dass der Verabreichungsvorgang beendet ist, also dass die gewünschte Dosis der medizinischen Substanz injiziert worden ist und dass die Kanüle oder Nadel herausgezogen werden kann. Das erzeugte Signal kann, wie oben erwähnt, zum Beispiel ein akustisches, optisches oder ein taktiles Signal sein. Das akustische Signal kann dabei beispielsweise ein Klickgeräusch, Piepston oder ein Klingeln sein. So kann zur Erzeugung eines solchen akustischen Signals der Signalerzeugungsmechanismus eine Feder beinhalten zum Bewegen von zwei Teilen zum Erzeugen eines Klickgeräusches oder eine Elektronik mit einem Lautsprecher zum Erzeugen eines Tons. Das optische Signal kann beispielsweise durch eine Farbänderung, Formänderung oder durch ein Erscheinen eines Anzeigeelements gebildet werden. Das taktile Signal kann in der Form eines für den Anwender spürbaren Vibrierens oder eines einmaliges Stosses realisiert sein. In diesem Fall kann der Signalerzeugungsmechanismus zum Beispiel einen mechanischen Schwingkreis mit einem Elektromagneten und einer Feder beinhalten. Ferner besteht auch die Möglichkeit, dass das Signal eine Kombination eines akustischen und eines taktilen Signals darstellt. In diesem Fall kann das Signal beispielsweise eine Vibration mit einem zusätzlichen Klickgeräusch umfassen.

Vorzugsweise verzögert die Kolbenstangeneinheit eine Drehbewegung der inneren Kolbenstange relativ zur äusseren Kolbenstangen. Wenn die innere Kolbenstange nach der Verzögerung eine Endposition erreicht hat, wird der Signalerzeugungsmechanismus ausgelöst.

Weiter betrifft die Erfindung eine Verabreichungsvorrichtung zur Verabreichung einer medizinischen Substanz umfassend ein Gehäuse, eine im Gehäuse angeordnete Einstechvorrichtung zum Einstechen einer Nadel oder Kanüle, einen Rückziehmechanismus zum Rückziehen der Nadel oder Kanüle in das Gehäuse oder in einen Medikamentenbehälter und eine Kolbenstangeneinheit nach einem der Ansprüche 1 bis 11. Dabei ist mit der Kolbenstangeneinheit am Ende eines Verabreichungsvorgangs das Zurückziehen der Nadel oder Kanüle mit dem Rückziehmechanismus verzögerbar.

Am Ende des Verabreichungsvorgangs verzögert die Kolbenstangeneinheit das Zurückziehen, so dass die Kanüle oder Nadel nicht unmittelbar nach dem Ausschütten der medizinischen Substanz herausgezogen wird. Dadurch kann die medizinische Substanz korrekt injiziert werden und die Gefahr einer Unterdosierung wird reduziert. Nach der Verzögerung löst die Kolbenstangeneinheit den Rückziehmechanismus aus. Das bedeutet, der Rückziehmechanismus zieht die Nadel oder Kanüle in das Gehäuse oder in den Medikamentenbehälter der Verabreichungsvorrichtung zurück, so dass die Nadel oder Kanüle aus der Einstechstelle herausgezogen wird.

Vorzugsweise verzögert die Kolbenstangeneinheit eine Drehbewegung der inneren Kolbenstange relativ zur äusseren Kolbenstangen. Wenn die innere Kolbenstange nach der Verzögerung eine Auslöseposition erreicht hat, wird die Nadel oder Kanüle durch den Rückziehmechanismus in proximaler Richtung aus der Einstechstelle herausgezogen.

In einer bevorzugten Ausführung ist die Verabreichungsvorrichtung ein Injektor. Beispiele für einen Injektor sind Autoinjektoren, Einweginjektoren und Mehrweginjektoren.

Unter den Begriff "Injektor" wird eine Vorrichtung verstanden, bei der die Injektionsnadel nach Abgabe der medizinischen Substanz aus dem Gewebe des Patienten entfernt wird. Somit verbleibt bei einem Injektor im Unterschied zu einem Infusionssystem die Injektionsnadel nicht permanent bzw. über einen längeren Zeitraum von mehreren Stunden im Patienten.

In einer bevorzugten Ausführung ist der Injektor als Einweginjektor ausgebildet. Ein Einweginjektor ist ein Injektor, welcher dazu verwendet wird, die in einer nicht nachfüllbaren und nicht austauschbaren Karpule befindliche medizinische Substanz zu injizieren. Ist die zur Injektion vorgesehene Menge der medizinischen Substanz in einem oder in mehreren Injektionsvorgängen injiziert worden, wird der Einweginjektor ausgewechselt. Die Karpule im Einweginjektor kann nicht ausgetauscht werden. Demgegenüber ist bei einem Mehrweginjektor die Karpule mit der medizinischen Substanz austauschbar. Wenn die erfindungsgemässe Kolbenstangeneinheit in einem Mehrweginjektor eingesetzt wird, wird das Fluidverdrängungselement vorzugsweise bei jedem Injektionsvorgang in eine zum vorhergehenden Injektionsvorgang andere Richtung bewegt. In einer anderen Ausführung kann das Fluidverdrängungselement im Mehrweginjektor bei jedem Injektionsvorgang ein Stück weit bewegt werden.

Alternativ kann es sich bei der Verabreichungsvorrichtung um ein Infusionssystem handeln, bei dem nach einem Verabreichungsvorgang mit der Kolbenstangeneinheit ein Endsignal verzögert wird. Ein Infusionssystem kann beispielsweise eine Patch-Pumpe oder eine Insulinpumpe sein.

Ferner betrifft die Erfindung ein Verfahren zur Herstellung einer Kolbenstangeneinheit für eine Verabreichungsvorrichtung zur Verabreichung einer medizinischen Substanz umfassend die Schritte
a. Bereitstellen einer zweistückigen äusseren Kolbenstange mit einem distalen, ersten Kolbenstangenteil, welches eine Kavität aufweist und einem zweiten Kolbenstangenteil, welches hülsenförmig ausgebildet ist;
b. Einfüllen eines dickflüssigen Fluids in die Kavität,
c. Einsetzen eines Fluidverdrängungselements in die Kavität, so dass sich mindestens ein Teil des Fluidverdrängungselements in das dickflüssige Fluid eintaucht, und montieren eines Dichtelements oder Schliessen einer Dichtung zwischen dem Fluidverdrängungselement und einer Innenwandung in der Kavität, so dass die Kavität fluidisch abgedichtet ist;
d. Verbinden des ersten Kolbenstangenteils mit dem zweiten Kolbenstangenteil, so dass das erste Kolbenstangenteil relativ zum zweiten Kolbenstangenteil in Rotationsrichtung und in Längsrichtung unbewegbar ist und wobei sich das Fluidverdrängungselement innerhalb des ersten Kolbenstangenteils und innerhalb des zweiten Kolbenstangenteils befindet.

Falls das Fluidverdrängungselement als einzelnes, separates Teil ausgebildet ist und nicht einteilig an der inneren Kolbenstangen ausgebildet ist, umfasst das Verfahren zudem die Schritte
e. koaxiales Einführen einer inneren Kolbenstange in die äussere Kolbenstange;
f. Verbinden der inneren Kolbenstange an einem distalen Ende der inneren Kolbenstange mit dem Fluidverdrängungselement, so dass die innere Kolbenstange relativ zum Fluidverdrängungselement in Rotationsrichtung und in Längsrichtung unbewegbar ist.

Dabei ist die Rotationsrichtung die Richtung um die Längsachse der äusseren Kolbenstange und die Längsrichtung ist die Richtung entlang der Längsachse der äusseren Kolbenstange.

In einer bevorzugten Ausführung erfolgt das Einfüllen des Fluids in die Kavität unter Vakuum oder Unterdruck. Bei einer Befüllung unter Vakuum oder Unterdruck können Einschlüsse von Luftbläschen vermieden oder reduziert werden.

### FIGUREN

- Fig. 1: zeigt eine Verabreichungsvorrichtung mit einer erfindungsgemässen Kolbenstangeneinheit in einer Schnittansicht, wobei der Schnitt durch die Längsachse der Verabreichungsvorrichtung verläuft;
- Fig. 2: zeigt eine Vergrösserung der Schnittansicht aus Fig. 1 in einem distalen Bereich der Kolbenstangeneinheit;
- Fig. 3: zeigt eine Explosionsansicht der Kolbenstangeneinheit; und
- Fig. 4: zeigt eine perspektivische Ansicht des ersten Kolbenstangenteils der Kolbenstangeneinheit.

### FIGURENBESCHREIBUNG

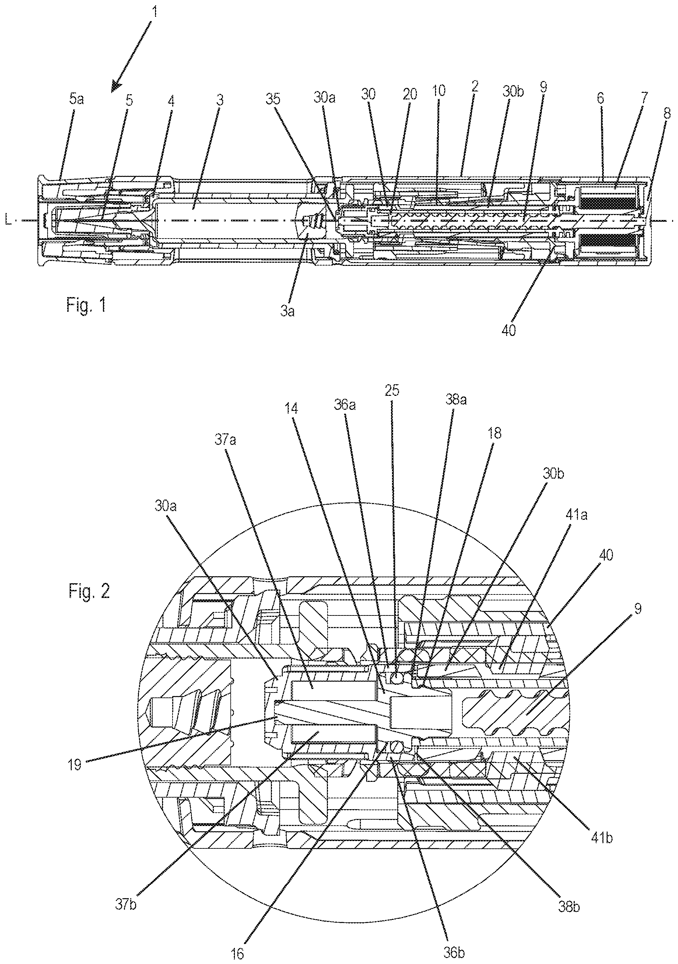

Figur 1 zeigt eine Verabreichungsvorrichtung in einer Schnittansicht, welche in Form eines Autoinjektors 1 ausgebildet ist. Der Schnitt verläuft dabei entlang einer mittigen Längsachse L des Autoinjektors 1.

Der Autoinjektor 1 weist ein hülsenförmiges, längliches Gehäuse 2 mit der Längsachse L auf. In diesem Gehäuse 2 sind ein Produktbehälter 3 mit der flüssigen medizinischen Substanz, einen Produktbehälterhalter, der als Spritzenhalter 4 bezeichnet wird, eine Kolbenstangeneinheit 10 mit einer äusseren Kolbenstange 30 und einer inneren Kolbenstange 20 und eine Antriebseinheit 6 angeordnet. An einem distalen Ende des Autoinjektors 1 weist dieser in seinem Auslieferungszustand eine Abziehkappe 5a auf, die vor der Verwendung des Autoinjektors 1 entfernt werden muss.

Nachfolgend werden die wichtigsten Bestandteile des Autoinjektors 1 kurz beschrieben. Eine ausführliche Beschreibung des Autoinjektors ist in der Patentanmeldung WO 2016/205962 A1 offenbart. Der in der WO 2016/205962 A1 beschriebene Autoinjektor weist, im Unterschied zur vorliegenden Erfindung keine Kolbenstangeneinheit 10 mit einer zweistückigen äusseren Kolbenstange auf, sondern umfasst eine einteilige äussere Kolbenstange.

Die Antriebseinheit 6 des Autoinjektors 1 umfasst eine spiralförmige Feder 7, die im Auslieferungszustand des Autoinjektors 1 mit so viel Energie vorgespannt ist, dass sie die im Produktbehälter 3 enthaltene medizinische Substanz mittels eines Ausschütthubs durch Verschieben eines Stopfens 3a aus dem Produktbehälter ausschütten kann. Die Antriebseinheit 6 umfasst weiter eine in Längsrichtung L ausgerichtete Gewindestange 9, welche in Gewindeverbindung mit der inneren Kolbenstange 20 der Kolbenstangeneinheit 10 ist. Die innere Kolbenstange 20 ist in Längsrichtung relativ zur äusseren Kolbenstange 30 gesichert, so dass bei einer Drehbewegung der Gewindestange 9 die innere 20 und die äussere Kolbenstange 30 in Längsrichtung zum distalen Ende relativ zum Gehäuse 2 bewegt werden. Die äussere Kolbenstange 30 umfasst einen distalen, ersten Kolbenstangenteil 30a und einen hülsenförmigen zweiten Kolbenstangenteil 30b, welcher proximal an den ersten Kolbenstangenteil 30a anschliesst. Der erste 30a und der zweite Kolbenstangenteil 30b sind in Rotations- und Längsrichtung so miteinander verbunden, dass sie relativ zueinander unbeweglich sind.

Der erste Kolbenstangenteil 30a weist an seinem distalen Ende eine Kontaktfläche 35 auf. Mit dieser wirkt der erste Kolbenstangenteil 30a mit einem Stopfen 3a des Produktbehälters 3 zusammen, um die medizinische Substanz aus dem Produktbehälter 3 auszuschütten. Der Spritzenhalter 1 ist relativ zum Gehäuse unbewegbar und mit diesem verrastet. Der Produktbehälter 3 ist in Bezug auf das Gehäuse 2 so angeordnet, dass die Nadelspitze einer Einstechnadel 5 distal über das distale Ende des Gehäuses 2 übersteht aber vor und nach der Injektion von einer Nadelschutzhülse abgedeckt wird. An seinem proximalen Ende weist das Gehäuse 2 eine Verschlusskappe 8 auf, die formschlüssig mit dem Gehäuse 2 dreh- und axialfest verbunden ist und das proximale Ende des Autoinjektors 1 bildet.

Zum Ausschütten der medizinischen Substanz aus dem Produktbehälter treibt die Antriebseinheit 6 die Gewindestange an, welche in Gewindeverbindung mit der inneren Kolbenstange 20 ist. Dadurch wird eine Axialbewegung erzeugt, wodurch die Kolbenstangeneinheit 10 den Stopfen in der Karpule in axialer Richtung verschieben kann. Die dabei auftretenden Axialkräfte werden von der inneren Kolbenstange 20 auf das Fluidverdrängungselement 14 übertragen. Über die Zentrierspitze 19 des Fluidverdrängungselements 14 werden die Axialkräfte weiter auf den ersten Kolbenstangenteil 30a übertragen, welcher die Kräfte über seine Kontaktfläche 35 auf den Stopfen überträgt.

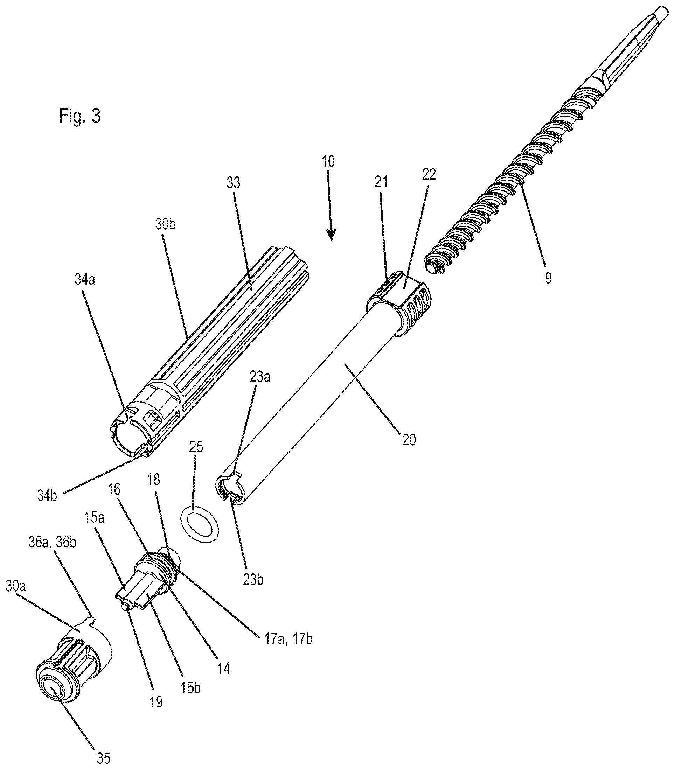

Nachfolgend wird die erfindungsgemässe Kolbenstangeneinheit 10 im Detail erläutert. Figur 3 zeigt eine Explosionsdarstellung der erfindungsgemässen Kolbenstangeneinheit 10. Sie umfasst die zweistückige äussere Kolbenstange, die innere Kolbenstange 20, ein mit der inneren Kolbenstange verbundenes Fluidverdrängungselement 14 und einen O-Ring 25, welcher zwischen dem Fluidverdrängungselement 14 und der inneren Kolbenstange 20 angeordnet ist.

Wie oben erwähnt, umfasst die äussere Kolbenstange 30 einen distalen, ersten Kolbenstangenteil 30a und einen proximal an den ersten Kolbenstangenteil 30a anschliessenden zweiten Kolbenstangenteil 30b. Der erste Kolbenstangenteil 30a hat im Wesentlichen eine zylindrische Form und weist eine Kavität auf.

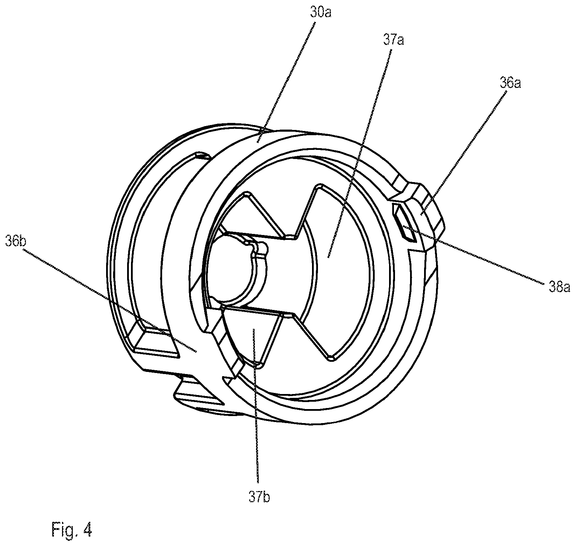

Figur 4 zeigt eine vergrösserte perspektivische Ansicht des ersten Kolbenstangenteils 30a. In dieser vergrösserten Darstellung ist ersichtlich, dass in der Kavität zwei Kammern 37a, 37b ausgebildet sind. Die Kammern 37a, 37b nehmen je ein Teilvolumen eines Zylinders ein und haben je im Querschnitt und in der Grundfläche die Form eines Kreissektors.

Der zweite Kolbenstangenteil 30b ist hülsenförmig ausgestaltet und weist im Wesentlichen den gleichen Aussendurchmesser auf wie der erste Kolbenstangenteil 30a. Der zweite Kolbenstangenteil 30b ist in einem Mechanikhalter 40 längsgeführt verdrehsicher gelagert. Hierzu sind auf einer Aussenfläche des zweiten Kolbenstangenteils 30b mehrere länglich ausgebildete und in Längsrichtung ausgerichtete Führungselemente 33 angeordnet, die sich über die fast über die ganze Länge des zweiten Kolbenstangenteils 30b erstrecken. Komplementär dazu sind auf einer Innenfläche des Mechanikhalters 40 Längsführungen angeordnet, in die die Führungselemente 33 eingreifen. Der Mechanikhalter 40 ist in Bezug auf das Gehäuse 2 entlang der Längsachse L unverschiebbar und drehfest angeordnet.

Wie in den Figuren 1 und 2 ersichtlich, ist die innere Kolbenstange 20 im zweiten Kolbenstangenteil 30b der äusseren Kolbenstange 30 gelagert. Die innere Kolbenstange 20 ist hülsenförmig ausgebildet und hat ein gegenüber dem restlichen Hülsenkörper leicht verdicktes proximales Ende. Dieses hat in etwa denselben Durchmesser wie die äussere Kolbenstange 30 und ist ausserhalb der äusseren Kolbenstange 30 angeordnet. Durch das verdickte Ende wird am proximalen Ende der inneren Kolbenstange 20 eine ringförmige Struktur 21 gebildet. Diese Struktur 21 wird durch zwei Nuten 22 unterbrochen. Die Nuten 22 haben zwei Funktionen. Die erste Funktion ist die Verdrehsicherung zwischen der inneren Kolbenstange 20 und dem Mechanikhalter 40 sicherzustellen, die zweite Funktion ist die Freigabe von Eingriffselementen 41a, 41b nach Abschluss der Produktausschüttung. Die innere Kolbenstange 20 weist an ihrem distalen Ende zwei sich gegenüberliegende Aussparungen 23a, 23b auf. In diese Aussparungen 23a, 23b greift je ein Nocken 17a, 17b des Fluidverdrängungselements 14 ein, so dass das Fluidverdrängungselement 14 drehfest mit der inneren Kolbenstange 20 verbunden ist.

Das Fluidverdrängungselement 14 weist in einem proximalen Bereich einen zylinderförmigen Abschnitt auf, welcher in den Innenraum der inneren Kolbenstange 20 ragt, ersichtlich in Figur 2. In diesem Abschnitt des Fluidverdrängungselements 14 sind die beiden Nocken 17a, 17b angeordnet. Zwischen den Nocken 17a, 17b erstreckt sich ein umlaufender Kragen 18, der im montierten Zustand formschlüssig in eine entsprechende Vertiefung im Innenraum der inneren Kolbenstange 20 eingreift. Das Fluidverdrängungselement 14 ist dadurch in Längsrichtung formschlüssig in der inneren Kolbenstange 20 gehalten. Zwischen diesem zylinderförmigen Abschnitt und einem distalen Bereich weist des Fluidverdrängungselements 14 einen mittleren Bereich mit einer umlaufende Nut 16 auf. In dieser Nut 16 ist ein O-Ring 25 aufgenommen. Der distale Bereich des Fluidverdrängungselements 14 weist zwei Flügel 15a, 15b auf, die als zwei rechteckförmige Elemente radial abstehen, wie in Figur 3 ersichtlich ist. An seinem distalen Ende umfasst das Fluidverdrängungselement 14 auf der Längsachse L eine zylindrische Zentrierspitze 19. Diese stützt das Fluidverdrängungselement 14 in radialer Richtung ab und stellt damit sicher, dass auch unter Einwirkung von Radialkräften das Fluidverdrängungselement 14 stets auf der Längsachse L ausgerichtet ist.

Im eingebauten Zustand befinden sich der distale und der mittlere Bereich des Fluidverdrängungselements 14 innerhalb der Kavität des ersten Kolbenstangenteils 30a der äusseren Kolbenstange 30 während der proximale Bereich (zylindrischer Abschnitt) des Fluidverdrängungselements 14 in der inneren Kolbenstange 20 aufgenommen ist. Diese Anordnung ist am besten in der vergrösserten Ansicht in Figur 2 ersichtlich. Erkennbar ist dort, dass der O-Ring 25 in der Nut 16 die Kavität abdichtet. Die zwei Flügel 15a, 15b des Fluidverdrängungselements 14 befinden sich je in einer Kammer 37a, 37b des ersten Kolbenstangenteils 30a. Durch die Ausformung der Kammer 37a, 37b kann sich das Fluidverdrängungselement 14 mit den Flügeln 15a, 15b um einen Winkel von mindestens 90° relativ zum ersten Kolbenstangenteil 30a drehen. In einer bevorzugten Ausführung ist das Fluidverdrängungselement 14 um einen Winkel von 110° relativ zum ersten Kolbenstangenteil 30a drehbar. Die Kammern 37a, 37b sind fast vollständig mit einem viskosen Fluid, insbesondere einer öligen, zähen, gelartigen oder pastösen Flüssigkeit befüllt. Vorzugsweise wird eine Silikonflüssigkeit mit einer kinematischen Viskosität zwischen 100'000 bis 500'000 Centistokes verwendet. Der erste Kolbenstangenteil 30a weist zwei in Längsrichtung auskragende Arme 36a, 36b auf, welche in entsprechende Ausnehmungen 34a, 34b im zweiten Kolbenstangenteil 30b eingreifen. Der erste Kolbenstangenteil 30a ist dadurch drehfest gegenüber dem zweiten Kolbenstangenteil 30b gesichert. Ferner weisen die Arme 36a, 36b an ihren Enden je einen radial nach innen weisenden Noppen 38a, 38b auf, die je eine Schulter am Fluidverdrängungselement 14 hintergreifen, so dass der erste Kolbenstangenteil 30a nicht in Längsrichtung relativ zum zweiten Kolbenstangenteil 30b bewegbar ist. Somit sind der erste und der zweite Kolbenstangenteil 30a, 30b relativ zueinander in Drehrichtung und in Längsrichtung unbewegbar gehalten.

Hingegen ist die innere Kolbenstange 20 zusammen mit dem Fluidverdrängungselement 14 relativ zur äusseren Kolbenstange 30 (und somit zum ersten 30a und zweiten Kolbenstangenteil 30b) drehbar, wenn die innere Kolbenstange 20 vom Mechanikhalter 40 freigegeben ist, wie nachfolgend erläutert.

Zu Beginn und während der Ausschüttung des Produkts aus dem Produktbehälter besteht eine Verdrehsicherung zwischen äusserer Kolbenstange 30, innerer Kolbenstange 20 und Mechanikhalter 40. Am Ende einer Ausschüttung, wenn die innere Kolbenstange 20 von proximal um etwa den Ausschütthub nach distal verschoben ist, wird die Verdrehsicherung der inneren Kolbenstange 20 gelöst, weil die Nuten 22 in diesem Zustand nicht mehr von Rippen des Mechanikhalters 40 geführt werden. Ein von der Antriebseinheit 6 über die Gewindestange 9 eingeleitetes Drehmoment induziert nun eine Drehung der inneren Kolbenstange 20 relativ zum Mechanikhalter 40 und zur äusseren Kolbenstange 30. Da das Fluidverdrängungselement 14 drehfest mit der inneren Kolbenstange 20 verbunden ist, dreht in diesem Fall das Fluidverdrängungselement 14 mit der inneren Kolbenstange 20. Der Drehung entgegen wirkt der durch das viskose Fluid hervorgerufene Widerstand. Dieser Widerstand verzögert die Rotation der inneren Kolbenstange 20, verhindert sie jedoch nicht, da bei einer Drehung des Fluidverdrängungselements 14 das Fluid in einem Spalt zwischen einer radialen Aussenfläche der Flügeln 15a, 15b und einer Innenfläche der Kammer von einer Seite der Flügel 15a, 15b zu anderen Seite fliessen kann.

Während der Drehung der inneren Kolbenstange 20 wirkt eine Axialkraft, welche von der Gewindestange 9 auf die Kolbenstangeneinheit 10 wirkt, weiterhin und sorgt dafür, dass während der Drehung der Stopfen des Produktbehälters 3 vollständig bis zu einem distalen Anschlag im Produktbehälter 3 gedrückt wird.

Hat sich die innere Kolbenstange 20 soweit gedreht, dass die Nuten 22 dieselbe Orientierung haben wie die Eingriffselemente 41a, 41b, wird ein Halteelement für eine axiale Bewegung in proximale Richtung freigegeben. Das vorgespannte Halteelement springt dadurch in proximaler Richtung zu einem Anschlag und erzeugt ein Klickgeräusch, welches dem Benutzer das Ende des Injektionsvorgangs anzeigt.

Die Eigenschaften der Kolbenstangeneinheit können durch die Form der Kavität, die Eigenschaften des Fluids sowie durch zusätzliche Elemente wie flügelartige, ruderartige oder teighakenartige Strukturen auf inneren Oberflächen des Fluidverdrängungselements und von innerer oder äusserer Kolbenstange beeinflusst werden.

### BEZUGSZEICHENLISTE

- 1: Autoinjektor
- 2: Gehäuse
- 3: Produktbehälter
- 3a: Stopfen
- 4: Spritzenhalter
- 5: Nadel
- 5a: Abziehkappe
- 6: Antriebseinheit
- 7: Feder
- 8: Verschlusskappe
- 9: Gewindestange

- 10: Kolbenstangeneinheit
- 14: Fluidverdrängungselement
- 15a, 15b: Flügel
- 16: Nut
- 17a,17b: Nocken
- 18: Kragen
- 19: Zentrierspitze
- 20: innere Kolbenstange
- 21: Struktur
- 22: Nut
- 23a, 23b: Ausnehmungen
- 25: Dichtelement

- 30: äussere Kolbenstange
- 30a: erster Kolbenstangenteil
- 30b: zweiter Kolbenstangenteil
- 33: Führungselemente
- 34a, 34b: Ausnehmungen

- 35: Kontaktfläche
- 36a, 36b: Arme
- 37a, 37b: Kammern
- 38a, 38b: Noppen

- 40: Mechanikhalter
- 41a, 41b: Eingriffselemente

## Patentansprüche

1. Kolbenstangeneinheit (10) für eine Verabreichungsvorrichtung (1) zur Verabreichung einer medizinischen Substanz, wobei mit der Kolbenstangeneinheit (10) ein Auslösen eines Mechanismus der Verabreichungsvorrichtung (1) verzögerbar ist, die Kolbenstangeneinheit (10) umfassend
a. eine äussere Kolbenstange (30) mit einer Kontaktfläche (35) an einem distalen Ende der äusseren Kolbenstange (30) zum Zusammenwirken mit einem Stopfen (3a) zum Ausschütten der Substanz aus einem Produktbehälter (3),
b. eine innere Kolbenstange (20), die derart in der äusseren Kolbenstange (30) gelagert ist, dass am distalen Ende der äusseren Kolbenstange (30) eine Kavität ausgebildet wird, welche zumindest teilweise mit einem dickflüssigen Fluid gefüllt ist,
c. ein Fluidverdrängungselement (14), welches sich mindestens teilweise in der Kavität befindet und mit der inneren Kolbenstange (20) zusammenwirkt oder einstückig mit der inneren Kolbenstange (20) ausgebildet ist und wobei das Fluidverdrängungselement (14) bei einer Verzögerungsbewegung relativ zur äusseren Kolbenstange (30) bewegbar ist,
d. wobei bei einer relativen Bewegung zwischen Fluidverdrängungselement (14) und äusserer Kolbenstange (30) durch Verdrängen des Fluids ein Bewegungswiderstand entsteht, so dass das Fluidverdrängungselement (14) eine den Mechanismus auslösende Auslöseposition verzögert erreicht,
**dadurch gekennzeichnet dass**,
die äussere Kolbenstange (30) zweistückig ausgebildet ist mit einem distalen, ersten Kolbenstangenteil (30a), welcher die Kontaktfläche (35) umfasst, die Kavität bildet, und mittels einer Dichtung und dem Fluidverdrängungselement (14) fluiddicht abgeschlossen ist, und einem an den ersten Kolbenstangenteil (30a) anschliessenden zweiten Kolbenstangenteil (30b).

2. Kolbenstangeneinheit (10) nach Anspruch 1, **dadurch gekennzeichnet, dass** die innere Kolbenstange (20) in Längsrichtung der Kolbenstangeneinheit (10) axialfest zur äusseren Kolbenstange (30) ist.

3. Kolbenstangeneinheit (10) nach Anspruch 1 oder 2, **dadurch gekennzeichnet, dass** die äussere Kolbenstange (30) derart ausgestaltet ist, dass mindestens die Hälfte der Länge der inneren Kolbenstangen (20) in der äusseren Kolbenstange (30) aufgenommen ist.

4. Kolbenstangeneinheit (10) nach einem der Ansprüche 1 bis 3, **dadurch gekennzeichnet, dass** das Fluidverdrängungselement (14) bei der Verzögerungsbewegung um eine Längsachse der äusseren Kolbenstange (30) relativ zur äusseren Kolbenstange (30) drehbar ist.

5. Kolbenstangeneinheit (10) nach Anspruch 4, **dadurch gekennzeichnet, dass** das Fluidverdrängungselement (14) um einen Winkel von mindestens 60°, vorzugsweise mindestens 90°, relativ zur äusseren Kolbenstange (30) drehbar ist.

6. Kolbenstangeneinheit (10) nach Anspruch 4 oder 5, **dadurch gekennzeichnet, dass** das Fluidverdrängungselement (14) einen Zapfen (19) aufweist zum Übertragen von axialen Kräften und zum Zentrieren des Fluidverdrängungselements (14) in der Kavität.

7. Kolbenstangeneinheit (10) nach einem der Ansprüche 1 bis 6, **dadurch gekennzeichnet, dass** das Fluidverdrängungselement (14) mindestens zwei Flügel (15a, 15b) aufweist zum Verdrängen des Fluids in der Kavität.

8. Kolbenstangeneinheit (10) nach Anspruch 7, **dadurch gekennzeichnet, dass** die Kavität eine erste Kammer (37a) aufweist zum Aufnehmen eines ersten der mindestens zwei Flügel (15a, 15b) und eine zweite Kammer (37b) aufweist zum Aufnehmen des zweiten der mindestens zwei Flügel (15a, 15b).

9. Kolbenstangeneinheit (10) nach einem der Ansprüche 1 bis 8, **dadurch gekennzeichnet, dass** das Fluid eine kinematische Viskosität zwischen 100'000 bis 500'000 Centistokes aufweist.

10. Kolbenstangeneinheit (10) nach einem der Ansprüche 1 bis 9, **dadurch gekennzeichnet, dass** das Fluidverdrängungselement (14) derart ausgebildet ist, dass zwischen dem Fluidverdrängungselement (14) und einer Innenfläche des ersten Kolbenstangenteils ein exklusiver Spalt besteht, so dass bei einer relativen Bewegung zwischen Fluidverdrängungselement (14) und äusserer Kolbenstange (30) das Fluid durch den Spalt fliesst.

11. Kolbenstangeneinheit (10) nach einem der Ansprüche 1 bis 10, **dadurch gekennzeichnet, dass** die Kavität fluiddicht ausgebildet ist, so dass das Fluid während der relativen Bewegung zwischen Fluidverdrängungselement (14) und äusserer Kolbenstange (30) in der Kavität verbleibt.

12. Verabreichungsvorrichtung (1) zur Verabreichung einer medizinischen Substanz umfassend einen Signalerzeugungsmechanismus zum Erzeugen eines Signals und eine Kolbenstangeneinheit (10) nach einem der Ansprüche 1 bis 11,
**dadurch gekennzeichnet, dass**
mit der Kolbenstangeneinheit (10) am Ende eines Verabreichungsvorgangs das Erzeugen eines Signals mit dem Signalerzeugungsmechanismus verzögerbar ist.

13. Verabreichungsvorrichtung (1) zur Verabreichung einer medizinischen Substanz umfassend ein Gehäuse (2), eine im Gehäuse (2) angeordnete Einstechvorrichtung zum Einstechen einer Nadel oder Kanüle, einen Rückziehmechanismus zum Rückziehen der Nadel oder Kanüle in das Gehäuse (2) oder in einen Medikamentenbehälter und eine Kolbenstangeneinheit (10) nach einem der Ansprüche 1 bis 11 **dadurch gekennzeichnet, dass**
mit der Kolbenstangeneinheit (10) am Ende eines Verabreichungsvorgangs das Zurückziehen der Nadel oder Kanüle mit dem Rückziehmechanismus verzögerbar ist.

14. Verfahren zur Herstellung einer Kolbenstangeneinheit (10) für eine Verabreichungsvorrichtung (1) zur Verabreichung einer medizinischen Substanz umfassend die Schritte
a. Bereitstellen einer zweistückigen äusseren Kolbenstange (30) mit einem distalen, ersten Kolbenstangenteil (30a), welcher eine Kavität aufweist und einem zweiten Kolbenstangenteil (30b), welcher hülsenförmig ausgebildet ist;
b. Einfüllen eines dickflüssigen Fluids in die Kavität;
c. Einsetzen eines Fluidverdrängungselements (14) in die Kavität, so dass mindestens ein Teil des Fluidverdrängungselements (14) in das dickflüssige Fluid eintaucht, und montieren eines Dichtelements (25) oder Schliessen einer Dichtung zwischen dem Fluidverdrängungselement (14) und einer Innenwandung in der Kavität, so dass die Kavität fluidisch abgedichtet ist;
d. Verbinden des ersten Kolbenstangenteils (30a) mit dem zweiten Kolbenstangenteil (30b), so dass der erste Kolbenstangenteil (30a) relativ zum zweiten Kolbenstangenteil (30b) in Rotationsrichtung und in Längsrichtung unbewegbar ist und wobei sich das Fluidverdrängungselement (14) innerhalb des ersten Kolbenstangenteils (30a) und innerhalb des zweiten Kolbenstangenteils (30b) befindet.

15. Verfahren nach Anspruch 14, **dadurch gekennzeichnet, dass** das Einfüllen des Fluids in die Kavität unter Vakuum oder Unterdruck erfolgt.

## Claims

1. A piston rod unit (10) for an administration device (1) for administering a medical substance, wherein the piston rod unit (10) can delay a release of a mechanism of the administration device (1), the piston rod unit (10) comprising:
a. an outer piston rod (30) with a contact face (35) at a distal end of the outer piston rod (30) for interacting with a stopper (3a) in order to discharge the substance from a product container (3)
b. an inner piston rod (20), which is mounted in the outer piston rod (30) in such a way that a cavity is formed at the distal end of the outer piston rod (30), said cavity being at least partially filled with a viscous fluid,
c. a fluid displacement element (14) which is located at least partially in the cavity and interacts with the inner piston rod (20) or is embodied integrally with the inner piston rod (20) and wherein the fluid displacement element (14) is moveable relative to the outer piston rod (30) during a delaying movement,
d. wherein during a relative movement between fluid displacement element (14) and outer piston rod (30) by a displacement of the fluid, a movement resistance occurs, such that the fluid displacement element (14) achieves a release position releasing the mechanism in delayed manner,
**characterized in that**
the outer piston rod (30) is configured in two parts with a distal, first piston rod part (30a), which comprises the contact face (35), forms the cavity, and is sealed in fluid-tight manner by means of a seal and the fluid displacement element (14) and a second piston rod part (30b) adjoining the first piston rod part (30a).

2. The piston rod unit (10) according to claim 1, **characterized in that** the inner piston rod (20) is axially fixed to the outer piston rod (30) in the longitudinal direction of the piston rod unit (10).

3. The piston rod unit (10) according to claim 1 or 2, **characterized in that** the outer piston rod (30) is configured such that at least the half of the length of the inner piston rod (20) is received in the outer piston rod (30).

4. The piston rod unit (10) according to any of claims 1 to 3, **characterized in that** during the delaying movement the fluid displacement element (14) is rotatable about a longitudinal axis of the outer piston rod (30) relative to the outer piston rod (30).

5. The piston rod unit (10) according to claim 4, **characterized in that** the fluid displacement element (14) is rotatable by an angle of at least 60°, preferably at least 90°, relative to the outer piston rod (30).

6. The piston rod unit (10) according to claim 4 or 5, **characterized in that** the fluid displacement element (14) has a pin (19) for transferring axial forces and for centering the fluid displacement element (14) in the cavity.

7. The piston rod unit (10) according to any of claims 1 to 6, **characterized in that** the fluid displacement element (14) has at least two wings (15a, 15b) for displacement of the fluid in the cavity.

8. The piston rod unit (10) according to claim 7, **characterized in that** the cavity has a first chamber (37a) for receiving a first of the at least two wings (15a, 15b) and has a second chamber (37b) for receiving the second of the at least two wings (15a, 15b).

9. The piston rod unit (10) according to any of claims 1 to 8, **characterized in that** the fluid has a kinematic viscosity between 100,000 to 500,000 centistokes.

10. The piston rod unit (10) according to any of claims 1 to 9, **characterized in that** the fluid displacement element (14) is configured such that an exclusive gap exists between the fluid displacement element (14) and an inner face of the first piston rod part, so that during a relative movement between fluid displacement element (14) and outer piston rod (30) the fluid flows through the gap.

11. The piston rod unit (10) according to any of claims 1 to 10, **characterized in that** the cavity is configured fluid-tight, so that the fluid remains in the cavity during the relative movement between fluid displacement element (14) and outer piston rod (30).

12. An administration device (1) for administering a medical substance comprising a signal generation mechanism for generating a signal and a piston rod unit (10) according to any of claims 1 to 11,
**characterized in that**
the generation of a signal with the signal generation mechanism can be delayed with the piston rod unit (10) at the end of an administration process.

13. The administration device (1) for administering a medical substance comprising a housing (2), an insertion device for inserting a needle or cannula arranged in the housing (2), a retraction mechanism for retracting the needle or cannula in the housing (2) or in a medicine container and a piston rod unit (10) according to any of claims 1 to 11,
**characterized in that**
with the piston rod unit (10) the retraction of the needle or cannula can be delayed with the retraction mechanism at the end of an administration process.

14. A method for manufacturing a piston rod unit (10) for an administration device (1) for administering a medical substance comprising the steps
a. Providing a two-part outer piston rod (30) with a distal, first piston rod part (30a), which has a cavity and a second piston rod part (30b), which is configured as a sleeve;
b. Filling a viscous fluid into the cavity;
c. Inserting a fluid displacement element (14) into the cavity, so that at least a portion of the fluid displacement element (14) immerses into the viscous fluid, and mounting a sealing element (25) or closing a seal between the fluid displacement element (14) and an inside wall in the cavity, so that the cavity is fluidically sealed;
d. Connecting the first piston rod part (30a) to the second piston rod part (30b), so that the first piston rod part (30a) is immovable relative to the second piston rod part (30b) in the rotational direction and the longitudinal direction and wherein the fluid displacement element (14) is located within the first piston rod part (30a) and within the second piston rod part (30b).

15. The method according to claim 14, **characterized in that** the filling of the fluid in the cavity occurs under vacuum or negative pressure.

## Revendications

1. Unité à tige de piston (10) pour un dispositif d'administration (1) pour l'administration d'une substance médicale, dans laquelle un déclenchement d'un mécanisme du dispositif d'administration (1) peut être retardé avec l'unité à tige de piston (10), l'unité à tige de piston (10) comprenant
a. une tige de piston extérieure (30) avec une surface de contact (35) à une extrémité distale de la tige de piston extérieure (30) pour la coopération avec un bouchon (3a) pour le versement de la substance à partir d'un contenant de produit (3),
b. une tige de piston intérieure (20), qui est montée dans la tige de piston extérieure (30) de telle sorte qu'une cavité, laquelle est remplie au moins en partie d'un fluide visqueux, est réalisée à l'extrémité distale de la tige de piston extérieure (30),
c. un élément de refoulement de fluide (14), lequel se trouve au moins en partie dans la cavité et coopère avec la tige de piston intérieure (20) ou est réalisé d'une seule pièce avec la tige de piston intérieure (20) et dans laquelle l'élément de refoulement de fluide (14) est mobile par rapport à la tige de piston extérieure (30) lors d'un mouvement de retardement,
d. dans laquelle lors d'un mouvement relatif entre l'élément de refoulement de fluide (14) et la tige de piston extérieure (30) à la suite du refoulement du fluide, une résistance au mouvement se produit, de sorte que l'élément de refoulement de fluide (14) atteint de manière retardée une position de déclenchement déclenchant le mécanisme,
**caractérisée en ce que**
la tige de piston extérieure (30) est réalisée en deux pièces avec une première partie de tige de piston (30a) distale, laquelle comprend la surface de contact (35), qui forme la cavité, et est fermée de manière étanche au fluide au moyen d'un joint et de l'élément de refoulement de fluide (14), et une deuxième partie de tige de piston (30b) raccordée à la première partie de tige de piston (30a).

2. Unité à tige de piston (10) selon la revendication 1, **caractérisée en ce que** la tige de piston intérieure (20) est solidaire axialement par rapport à la tige de piston extérieure (30) dans la direction longitudinale de l'unité à tige de piston (10).

3. Unité à tige de piston (10) selon la revendication 1 ou 2, **caractérisée en ce que** la tige de piston extérieure (30) est conçue de telle sorte qu'au moins la moitié de la longueur de la tige de piston intérieure (20) est reçue dans la tige de piston extérieure (30).

4. Unité à tige de piston (10) selon l'une quelconque des revendications 1 à 3, **caractérisée en ce que** l'élément de refoulement de fluide (14) lors d'un mouvement de retardement peut tourner par rapport à la tige de piston extérieure (30) autour d'un axe longitudinal de la tige de piston extérieure (30) .

5. Unité à tige de piston (10) selon la revendication 4, **caractérisée en ce que** l'élément de refoulement de fluide (14) peut tourner par rapport à la tige de piston extérieure (30) d'un angle d'au moins 60°, de préférence au moins 90°.

6. Unité à tige de piston (10) selon la revendication 4 ou 5, **caractérisée en ce que** l'élément de refoulement de fluide (14) présente un tourillon (19) pour la transmission de forces axiales et pour le centrage de l'élément de refoulement de fluide (14) dans la cavité.

7. Unité à tige de piston (10) selon l'une quelconque des revendications 1 à 6, **caractérisée en ce que** l'élément de refoulement de fluide (14) présente au moins deux ailettes (15a, 15b) pour le refoulement du fluide dans la cavité.

8. Unité à tige de piston (10) selon la revendication 7, **caractérisée en ce que** la cavité présente une première chambre (37a) pour la réception d'une première des au moins deux ailettes (15a, 15b) et une deuxième chambre (37b) pour la réception de la deuxième des au moins deux ailettes (15a, 15b).

9. Unité à tige de piston (10) selon l'une quelconque des revendications 1 à 8, **caractérisée en ce que** le fluide présente une viscosité cinématique comprise entre 100 000 et 500 000 centistokes.

10. Unité à tige de piston (10) selon l'une quelconque des revendications 1 à 9, **caractérisée en ce que** l'élément de refoulement de fluide (14) est réalisé de telle sorte qu'une fente exclusive est présente entre l'élément de refoulement de fluide (14) et une surface intérieure de la première partie de tige de piston, de sorte que lors d'un mouvement relatif entre l'élément de refoulement de fluide (14) et la tige de piston extérieure (30), le fluide s'écoule à travers la fente.

11. Unité à tige de piston (10) selon l'une quelconque des revendications 1 à 10, **caractérisée en ce que** la cavité est réalisée de manière étanche au fluide, de sorte que le fluide reste dans la cavité pendant le mouvement relatif entre l'élément de refoulement de fluide (14) et la tige de piston extérieure (30).

12. Dispositif d'administration (1) pour l'administration d'une substance médicale comportant un mécanisme de génération de signal pour la génération d'un signal et une unité à tige de piston (10) selon l'une quelconque des revendications 1 à 11,
**caractérisé en ce que**
la génération d'un signal avec le mécanisme de génération de signal peut être retardée avec l'unité à tige de piston (10) à la fin d'un processus d'administration.

13. Dispositif d'administration (1) pour l'administration d'une substance médicale comportant un logement (2), un dispositif d'enfoncement pour l'enfoncement d'une aiguille ou canule disposé dans le logement (2), un mécanisme de retrait pour le retrait de l'aiguille ou la canule dans le logement (2) ou dans un contenant de médicament et une unité à tige de piston (10) selon l'une quelconque des revendications 1 à 11
**caractérisé en ce que**
le retrait de l'aiguille ou la canule avec le mécanisme de retrait peut être retardé avec l'unité à tige de piston (10) à la fin d'un processus d'administration.

14. Processus pour fabriquer une unité à tige de piston (10) pour un dispositif d'administration (1) pour l'administration d'une substance médicale comprenant les étapes
a. de fourniture d'une tige de piston extérieure (30) en deux pièces avec une première partie de tige de piston (30a) distale, laquelle présente une cavité, et une deuxième partie de tige de piston (30b), laquelle est réalisée en forme de manchon ;
b. de versement d'un fluide visqueux dans la cavité ;
c. d'insertion d'un élément de refoulement de fluide (14) dans la cavité, de sorte qu'au moins une partie de l'élément de refoulement de fluide (14) plonge dans le fluide visqueux, et de montage d'un élément d'étanchéité (25) ou de fermeture d'un joint entre l'élément de refoulement de fluide (14) et une paroi intérieure dans la cavité, de sorte que la cavité est étanchéifiée de manière fluidique ;
d. d'assemblage de la première partie de tige de piston (30a) à la deuxième partie de tige de piston (30b), de sorte que la première partie de tige de piston (30a) est immobile dans le sens de rotation et dans la direction longitudinale par rapport à la deuxième partie de tige de piston (30b) et dans lequel l'élément de refoulement de fluide (14) se trouve à l'intérieur de la première partie de tige de piston (30a) et à l'intérieur de la deuxième partie de tige de piston (30b).

15. Procédé selon la revendication 14, **caractérisé en ce que** le versement du fluide dans la cavité s'effectue sous vide ou dépression.
